(19) Europäisches Patentamt
European Patent Office
Office européen des brevets

(11) EP 2 431 738 A1

(12) **EUROPEAN PATENT APPLICATION**
published in accordance with Art. 153(4) EPC

(43) Date of publication:
**21.03.2012 Bulletin 2012/12**

(21) Application number: **09844700.6**

(22) Date of filing: **01.12.2009**

(51) Int Cl.:
***G01N 33/15*** *(2006.01)* ***G01N 33/68*** *(2006.01)*
***G01N 33/52*** *(2006.01)*

(86) International application number:
**PCT/KR2009/007114**

(87) International publication number:
**WO 2010/131826 (18.11.2010 Gazette 2010/46)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **12.05.2009 KR 20090041235**

(71) Applicant: **Innopharmascreen Inc.**
**Asan-si, Chungcheongnam-do 336-851 (KR)**

(72) Inventors:
- **KANG, In-Cheol**
  **Seoul 135-796 (KR)**
- **KIM, Eung-Yoon**
  **Asan-si**
  **Chungcheongnam-do 336-851 (KR)**
- **CHOI, Young-Jin**
  **Asan-si**
  **Chungcheongnam-do 336-851 (KR)**

(74) Representative: **Richter Werdermann Gerbaulet Hofmann**
**Patentanwälte**
**Neuer Wall 10**
**20354 Hamburg (DE)**

(54) **METHOD FOR SCREENING OSTEOPONTIN INHIBITOR AND INHIBITOR PREPARED THEREBY**

(57) Provided is a compound found by screening a material for inhibiting an osteopontin, in which the material for inhibiting the osteopontin can increase effectiveness as an excellent material for inhibiting an osteoporosis.

[Fig. 1]

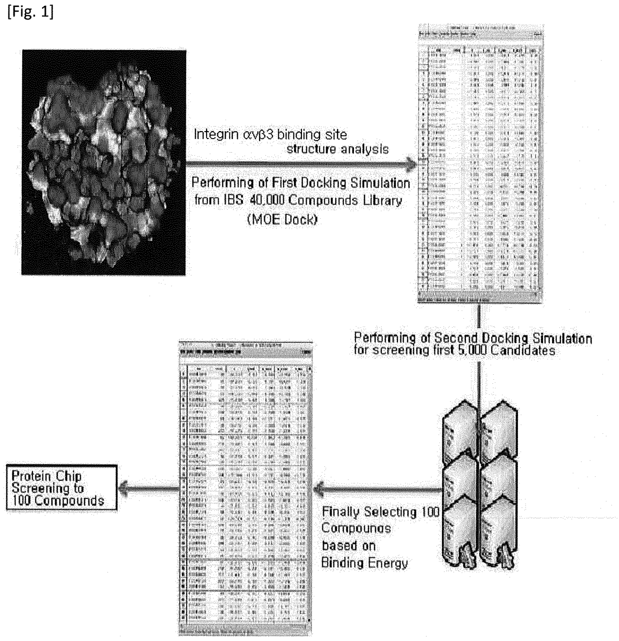


EP 2 431 738 A1

## Description

## BACKGROUND OF THE INVENTION

### Field of the Invention

The present invention relates to a method for screening an osteopontin inhibitor, and an inhibitor obtained from the method.

### Description of Related Art

It is known that an osteopontin is produced in osteoclast and osteoblast, and plays an important role in the born metabolism. It is not required for normal born generation in mice, but is involved in the bone regeneration, and sometimes acts as a defense mechanism against an inflammatory and a wound. In addition, the studies have been reported that it has the activities for promoting growth and metastasis of cancer cell. A reduction of bone density and a deterioration of osseous tissue rarely occur in the mice without an osteopontin (Hiroyuki Yoshitake et al., 1999, PNAS., 96:8156-8160), and in the case of the mice that have symptoms of arthritis and an osteopontin that normally acts, the symptoms are gradually deteriorated, the joints become swollen, and the cartilages are destructed. On the other hand, the symptoms of arthritis with a suppressed osteopontin are not processed and the cartilages are not destructed (Kenji Yumoto et al., 2002 PNAS., 99:4556-4561). From these results, it can be known that the materials for suppressing the activity of osteopontin can be used for treating and preventing an osteoporosis, a rhemarthritis, and a periodontal disease.

An osteopontin is glycoprotein, has a mass of 44 Kilo Dalton, has a phosphorylated serine and threonine, and has acidity. The feature of the osteopontin can be explained that it has the ability to bind with much calcium because it has a calcium-binding motif (Giachelli, C. M et al., 1995 Trends Cardiovasc Med 3:88-95). The osteopontin in born matrix stimulates to attach osteoclast to born through a substrate binding (Ross, F.P., et al., 1993 J. Biol. Chem. 268:9901-9907). A water-soluble osteopontin in osteoclast controls a level of calcium, acts as a chemotaxis material, and suppresses a death of endothelioid cells (Khan SA., et al., 2002 Mol. Biol. Cell 85:728-736).

The osteopontin has a domain including RGD (Arginine-Glycine-Aspartate) so that it interacts with integrins ($\alpha v\beta 3$, $\alpha v\beta 1$, $\alpha v\beta 5$) on cell surface; involves in cell adhesion; and the sequence (SVVYGLR) interacts with integrins ($\alpha 9\beta 31$, $\alpha 4\beta 1$) to exposure to a thrombin cleavage. And, various CD44 variants are known as a receptor of the osteopontin (Giachelli C. M., et al., 2000 Matrix biology 19:615-622).

$\alpha v\beta 3$ that is a main integrin on the surface of osteoclast expresses at a low level in small intestine and blood vessel myocytes, and the like, but expresses at a high level in born, an inflammation region, placenta, and an infiltrating tumor to act on born re-absorption of osteoclast, an activation of microphage, an angiogenesis, and the like. Especially, the osteopontin acts as integrin $\alpha v\beta 3$ ligand to increase born reabsorption by osteoclast so that it stimulates the movement of osteoclast and involves the initial adherence (Ichiro Nakamura., et al., 2007 J Bone Miner Metab 25:337-344). It is known that the sequence RGD of osteopontin mediates the binding between osteopontin and integrin $\alpha v\beta 3$ for cell adherence, and also minerals, such as Ca, Mn, Mg, and the like play an important role (Dana D. Hu., et al., 1995 JBC 28:9917-9925).

Osteoclast is differentiated from hematopoietic stem cell (Boyle WJ., et al., 2003 Nature 423:337-42). It has been found that osteoclast is normally generated in the mice with a suppressed osteopontin expression for the differentiation of osteoclast, but the differentiation of osteoclast is suppressed at an initial differentiation of osteoclast by RANKL in the differentiation of human osteoclast with a reduced osteopontin expression, and as a result, it has been raised that osteopontin is an important factor for differentiating to osteoclast in human (Cathy J. Aitken., et al., 2004 J. Cell. Biochem 93:896-903).

## SUMMARY OF THE INVENTION

The present invention has been invented due to the needs as mentioned above, and it is, therefore, an object of the present invention to provide a method for screening an osteopontin inhibitor.

It is another object of the present invention to provide an osteopontin inhibitor.

It is another object of the present invention to provide use of an osteopontin inhibitor for treating diseases.

In accordance with one aspect of the present invention to achieve the object, there is provided a method for screening an osteopontin inhibitor, comprising: a) screening a starting material for a protein chip screening by performing a molecular docking simulation to the whole files of compound library; b) preparing a mixture by mixing the screened starting material with an osteopontin, and a fluorescent material-marked osteopontin; c) adding the mixture on an integrin $\alpha v\beta 3$ receptor fixed to the protein chip; and d) analyzing the degree of binding.

The screening method of the present invention is called 'Integrated Drug Discovery Platform (IDDP) Technique.'

The integrated drug discovery platform technique is a creative new drug development system of InnoPharmaScreen, that is a standard platform of new drug development for discovering most effective drug candidate materials with the minimal cost within the earliest time from a target discovering to an optimization of leading material integrately using Structure-based Screening *(in silico*), Protein Chip-based Screening (HTS), and/or Cell-based Assay *(in vitro*).

In addition, the integrated drug discovery platform technique is an integrated new drug discovering system for IT/BT/NT that is certainly different from the traditional new drug discovering system. Therefore, it has the following advantages: it can dramatically decrease the amount of time from the target analysis to the optimization of leading materials to a minimum of three months to not more than one year, which generally requires the time of at least three years; the assays of main targets, such as Kinase, GPCR, Protease, Phosphatase, and the like are set up; the assay of each target for verifying a medicinal effect is ready to start; the sensitivities of successful leading materials are guaranteed because $IC_{50}$ of the leading materials is not more than 1 uM; the progresses to a pre-clinical stage and clinical stage are handled very efficiently because various data about the screened materials are provided through each of steps for the integrated new drug discovering; the materials selected from the structure-based screening are verified *in vitro* because they go through the chip-based High-Throughput-Screening, again; the correlationship between a minority of the successful materials can be correctly investigated through the structure-based molecular simulation again; and also the cost required for new drug development is dramatically decreased by screening *in silico* and *in vitro* at the same time.

For one specific example of the present invention, a search algorithm for the docking calculations preferably uses an alpha triangle method, but not limited thereto.

For one specific example of the present invention, the fluorescent material is preferably at least one fluorescent material selected from the group consisting of Cy3 (Green), Cy5 (Red), FITC (Green), Alexa, BODIPY, Rhodamine, and Q-dot, and more preferably, Cy5, but not limited thereto.

The present invention provides an osteopontin inhibitor including at least one compound selected from the group consisting of the following Chemical Formula 1 to Chemical Formula 3, a derivative thereof, and salt thereof:

[Chemical Formula 1]

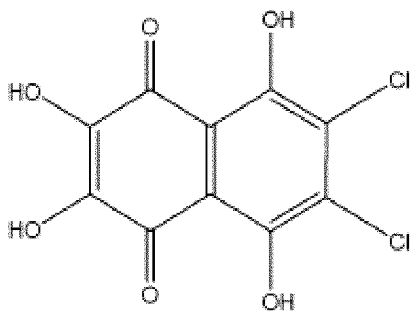

(IPS-02001) :
6,7-Dichloro-2,3,5,8-tetrahydroxy-[1,4]naphthoquinone

[Chemical Formula 2]

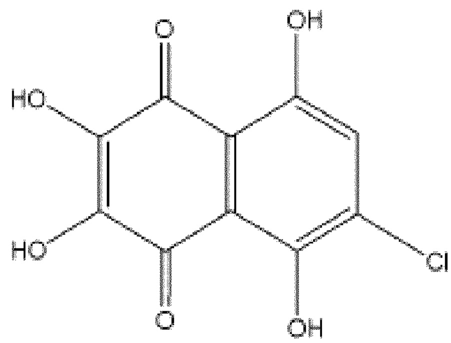

(IPS-02002) : 6-Chloro-2,3,5,8-tetrahydroxy-[1,4]naphthoquinon

[Chemical Formula 3]

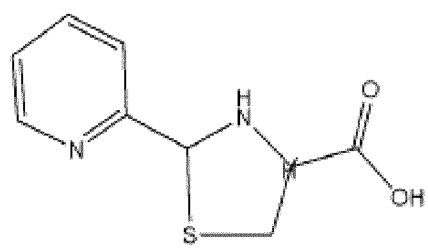

(IPS-02003) : 2-Pyridin-2-yl-thiazolidine-4-carboxylic acid

In addition, the present invention provides a composition for treating or preventing an osteoporosis, a rhemarthritis, and a periodontal disease, in which the composition includes at least one compound selected from the group consisting of the above Chemical Formula 1 to Chemical Formula 3, a derivative thereof, and salt thereof.

The term, 'derivative' used in the present invention means a similar compound obtained by chemically changing a part of certain compound, and for example, the compound, in which hydrogen atom or specific atomic group of the compound is substituted with other atom or atomic group.

The compound according to the present invention is an osteopontin inhibitor that can be used for inhibiting the osteopontin activity.

Therefore, a pharmaceutical formulation including a proper compound or salt thereof as well as one compound of Chemical Formula 1 to Chemical Formula 3, a derivative thereof and/or salt thereof according the present invention may be used for treating a disease that needs the inhibition of osteopontin.

For example, the disease includes the inflammatory of joint including a rhemarthritis and a rheumatoid arthritis, as well as other joint disease, such as a rheumatoid spondylitis and osteoarthritis. The additional application possibility is to treat an osteoporosis, a periodontal disease, and the like.

In the case of a drug formulation, a general adjuvant, a carrier, and an additive are used in addition to an effective amount of the compound according to the present invention, or salt thereof.

The dosages of active components may depend on the administration way, age of patient, weight of patient, the feature of the disease to be treated, a severity of the disease to be treated, and the similar factors of the disease to be treated.

One-day dosage may be administrated in a single dosage that is administrated once a day, or may be dividedly administrated in at least two a day, and generally may be 0.001 to 100 mg.

The preferable administration form includes an oral, a parenteral, a percutaneous, a topical, an inhalation, and an intranasal formulation.

The general pharmaceutical form of the formulation may be used, and for example, include a tablet, a coated tablet, a capsule, dispersive powders, a granule, an aqueous liquid form, aqueous and oil suspensions, syrups, a solvent, or drops.

The drug in a type of solid may include inactivated component and a carrier, for example, calcium carbonate, calcium phosphate, sodium phosphate, lactose, starch, mannitol, alginate, gelatin, guar gum, magnesium or aluminum stearate, methylcellulose, talc, high dispersible silica, silicon oil, high molecular weight fatty acid (For example: stearic acid), agar agar, vegetable or animal fat and oil, and high molecular weight polymer (For example: polyethylene glycol), and the formulation suitable for the oral administration may include any additional flavoring agent and/or sweetening agent.

The drug in a type of liquid may be sterilized and/or may include an adjuvant, such as a preservative, a stabilizer, a wetting agent, a permeable agent, an emulsifier, a dispersing agent, a solubilizer, salt, sugar or sugar alcohol, and/or a viscosity agent, for controlling or buffering an osmotic pressure.

Example of the additive as mentioned above includes tartrate and citrate buffer, ethanol, a complexing agent (For example: ethyleneaminetetraacetic acid and non-toxic salt thereof). High molecular weight polymer, such as liquid polyethylene oxide, microcrystalline cellulose (For example: carboxymethylcellulose), polyvinylpyrrolidone, dextrane or gelatin, is considered in order to control the viscosity. For example, starch, lactose, mannitol, methylcellulose, talc, high dispersive silica, high molecular weight fatty acid (For example: stearic acid), gelatin, agar agar, calcium phosphate, magnesium stearate, animal and vegetable fat, and solidified high molecular weight polymer (For example: polyethylene glycol) are used as solid carrier material.

A parenteral or topical oil suspension may include vegetable, synthesized or semi-synthesized oil, for example, a liquid fat ester with 8 to 22 carbon atom at chain of fatty acid (For example: palmitic acid, lauric acid, tridecylic acid, margaric acid, stearic acid, arachidic acid, myristic acid, behenic acid, pentadecylic acid, linolenic acid, elaidic acid, brassidic acid, erucic acid, or oleic acid) that is esterificated with primary alcohol or tertiary alcohol (For example: methanol, ethanol, propanol, butanol, pentanol or isomer thereof, glycol, or glycerol) with 1 to 6 carbon atom respectively. Example of the fat ester as mentioned above includes commercial Myglyol, isopropyl myristate, isopropyl palmitate, isopropyl stearate, PEG 6-capric acid of saturated fat alcohol, caprylic acid/capric acid ester, polyoxyethylene glycerol trioleate, ethyl oleate, wax fat ester (For example: Synthesized duck rump gland fat), isopropyl ester of coconut oil fatty acid, oleyl oleate, decyl oleate, ethyl lactate, dibutyl phthalate, diisopropyl adipate, fatty acid ester of polyol, and the like. Silicon oils or fat alcohols with different viscosities (For example: isotridecyl alcohol, 2-octyl dodecanol, cetyl stearyl alcohol or oleyl alcohol) and fatty acid (For example: oleic acid) are also preferably used. In addition, vegetable oils, such as a castor oil, almond oil, an olive oil, sesame oil, a cottonseed oil, a peanut oil, or soybean oil, can be used.

A gelator and a solubilizer, water, or the solvent compatible with water are considered as an example of the solvent. Example of the solvent includes alcohol, such as ethanol or isopropanol, benzyl alcohol, 2-octyldodecanol, polyethylene glycol, phthalate, adipate, propylene glycol, glycerin, dipropylene glycol, tripropylene glycol, wax, methyl cellosolve, cellosolve, ester, morpholine, dioxane, dimetylsulfoxide, dimethylformamide, tetrahydrofuran, cyclohexanone, and the like.

Cellulose ether that is possible to dissolve or swell in an organic solvent (For example: hydroxypropylmethylcellulose, methylcellulose, ethylcellulose, or soluble starch) as well as water may be used as a film former.

A mixed form of the gelator and the film former may be possile to be used. Most of all, the present invention may use an ionic macromolecule, and for example, the ionic macromolecule may be sodium carboxymethylcellulose, polyacrylic acid, polymethacrylic acid and salt thereof, sodium amylopectin semiglycolate, alginic acid, or propylene glycol alginate as sodium salt, arabic gum, xanthan gum, guar gum, or carrageenan gum.

An additional formulation adjuvant may include glycerin, paraffins with different viscosities, triethanolamine, collagen, allantoin, and novantrone. The wetting agent, the emusifier, or a surfactant, such as sodium lauryl sulfate, fat alcohol ether sulfate, disodium N-lauryl-β-imino dipropionate, polyethoxylated castor oil, or sorbitan monooleate, sorbitan monostearate, polysorbate (For example: Tween), cetyl alcohol, lecithin, glycerin monostearate, polyoxyethylene stearate, alkylphenol polyglycol ether, cetyltrimethylammonium chloride, or monoalkyl or dialkyl polyglycol ether orthophosphate monoethanolamine salt. The stabilizer for stabilizing latex (For example: montmorillonite or colloidal silica), the stabilizer for preventing the degradation of active material, such as an antioxidant (For example: tocopherol or butylhydroxyanisol), or a preservative (For example: p-hydroxybenzoate ester) may be also required for preparing the desired formulation.

The formulation for the parenteral administration may be presented in a type of an individual administrating unit, such as, an ample or a bial. Preferably, a solution of active component, especially, an aqueous solution, and most of all, an isotonic solution are used, and also a suspension is used. The above injection type may be used as a finished formulation, or the active compound, such as a freeze-dried material may be prepared just before the use by mixing it with other solid carrier material, the desired solvent, or the suspension.

The intranasal formulation may be presented in an aqueous or oil solution, or an aqueous or oil suspension. It may be also the freeze-dried material that is prepared in a proper solvent or suspension before the use.
The preparing of the formulation, the filling of the formulation to a container, and the sealing of the container are performed under the general antibacterial and aseptic conditions.
The compounds according to the present invention may be prepared by using the preparation methods that are generally known in the art.
For one specific example of the present invention, the compounds according to the present invention are screened by using Natural Compound Library (40,000 species) commercially available from the company, that is, InterBioScreen(IBM) (www.ibscreen.com) in Russia, and purchased from the company, InterBioScreen(IBM) in Russia.
Hereinafter, the present invention will be described.
An osteopontin is glycoproten that is abundantly presented in a born matrix, and involved in the adhesion, the migration, and the differentiation of osteoclast, in which the osteoclasat is the cause of the disease related to born, such as an osteoporosis, a rheumatoid arthritis, a periodontal disease, and the like. The material that inhibits the activity of the osteopontin may be developed as a raw material of the therapeutic agent and medical substance for the disease related to born as mentioned above. In order to screening the osteopontin inhibitor, the method for easily and quickly screening ten thousands of the compounds is needed. The materials screened by using a protein chip and virtual screening process may be developed as the osteopontin inhibitor.
The material for inhibiting the activity of osteopontin according to the screening method of the present invention may be developed as a raw material of the therapeutic agent and medical substance for the disease related to born by suppressing the differentiation and migration of osteoclast.

## BRIEF DESCRIPTION OF THE DRAWINGS

The accompanying drawings are included to provide a further understanding of the present invention, and are incorporated in and constitute a part of this specification. The drawings illustrate exemplary embodiments of the present invention and, together with the description, serve to explain principles of the present invention. In the drawings:
Fig. 1 shows a method for the virtual screening process of the osteopontin inhibitor, and Table 1 shows the binding energy values of 100 compounds that have most excellent binding strength as a result of the virtual screening.
Fig. 2 is the experiment result and graph for identifying the binding generated by the interaction between the osteopontin and integrin $\alpha v\beta 3$ per the concentration.
Fig. 3 is a representative experiment result and virtual image thereof of the binding generated by the interaction between the osteopontin and integrin $\alpha v\beta 3$ from Compound Library derived from natural substances.
Fig. 4 shows the experiment result and inhibition concentration (IC50; half-maximal inhibition concentration) that are depended on the concentration as a competitive inhibitor of binding between the osteopontin and integrin $\alpha v\beta 3$.
Fig. 5 shows the chemical structure and name thereof of the material that inhibits the osteopontin.
Fig. 6 shows the results for analyzing the ability of the screened inhibition material of the osteopontin for suppressing the differentiation of osteoclast using biological effect analysis.
Fig. 7 shows the experiment results showing the effect on suppressing the born destruction using *in vivo* Calvaria animal model.
Fig. 8 shows the charted graph showing the destructed born area of *in vivo* Calvaria animal model from the result of Fig. 7.

## DESCRIPTION OF specific embodiments

Hereinafter, the present invention will be described in more detail with reference to the following Examples. However, the following Examples are only for understanding the present invention, but the range of the present invention is not limited thereto in a certain sense.
<u>Example 1: Virtual Screening for Material that interrupts Binding of Osteopontin and Integrin</u>
The software used for a virtual screening of Fig. 1 was MOE (Molecular Operating Environment) program of Chemical Computing Group. The method for screening used a molecular docking simulation. The specific method was as follows. Firstly, a primary molecular docking simulation was performed to the whole files of 40,000 compounds library. As a screening algorithm for a docking calculation, a structure change energy calculation was performed in the maximum 500,000 times per each ligand compound using Alpha Triangle Method. The method used the algorithm for docking by giving shape to three points of the molecular as a triangle and then measuring as to whether the receptor protein matches with another triangle. LondondG method was used as the scoring method to calculate the maximum 10 pose per ligand. There are three types, that is, LondondG, AffinitydG, and AlphaHB as the scoring method supported from MOE, and LondondG used in the calculation was as follows:
$$\Delta G = c + E_{flex} + \sum_{h-bonds} c_{HB} f_{HB} + \sum_{m-lig} c_M f_M + \sum_{atoms\ i} \Delta D_i$$

As LondondG function, the change of rotational/translation entropy due to the binding, the reduction of flexibility energy due to the binding of ligand, the hydrogen binding energy, the metal ion ligation, the difference of desolavtion energy, and the like, were used as the parameters.

5,000 compounds with an excellent binding strength were selected from the primary docking simulation results of 40,000 compounds, and then secondary docking simulation was performed with the above 5,000 compounds. The method used for the secondary docking simulation was basically the same with that of the primary docking simulation, but was further added with the method for minimizing energy. The most stable ligand structure that can be from the binding structure was screened by performing energy minimized molecular mechanics calculation to the docked ligand pose. As a result, the calculated binding energy value was used as the parameter for screening the binding strength. As a result of the docking simulation and energy minimization to 5,000 compounds, 100 compounds with the most excellent LondondG value that was a score were selected to suggest 100 compounds as a starting material for the protein chip screening (see Table 1).

[Table 1]

| Number | ID | Energy | Number | ID | Energy | Number | ID | Energy | Number | ID | Energy | Number | ID | Energy |
|---|---|---|---|---|---|---|---|---|---|---|---|---|---|---|
| 1 | 30579 | -316.11 | 21 | 61012 | -273.51 | 41 | 52579 | -253.41 | 61 | 66971 | -246.24 | 81 | 40546 | -237.46 |
| 2 | 3842 | -314.83 | 22 | 923 | -271.64 | 42 | 11597 | -252.70 | 62 | 49637 | -246.20 | 82 | 28110 | -237.04 |
| 3 | 4276 | -302.77 | 23 | 21249 | -271.45 | 43 | 7173 | -252.46 | 63 | 66869 | -245.96 | 83 | 20025 | -236.90 |
| 4 | 6756 | -294.35 | 24 | 4022 | -270.59 | 44 | 65924 | -251.61 | 64 | 44253 | -245.45 | 84 | 51544 | -236.38 |
| 5 | 69872 | -294.16 | 25 | 51068 | -269.74 | 45 | 60989 | -251.42 | 65 | 54702 | -245.27 | 85 | 54345 | -236.34 |
| 6 | 3792 | -289.08 | 26 | 37527 | -268.28 | 46 | 56104 | -251.35 | 66 | 27136 | -245.06 | 86 | 62408 | -235.26 |
| 7 | 44176 | -288.57 | 27 | 7569 | -266.11 | 47 | 69334 | -250.93 | 67 | 72149 | -244.93 | 87 | 53428 | -235.07 |
| 8 | 9237 | -286.54 | 28 | 57396 | -264.47 | 48 | 52945 | -250.86 | 68 | 58303 | -244.80 | 88 | 17322 | -234.58 |
| 9 | 18946 | -286.42 | 29 | 36177 | -262.62 | 49 | 50559 | -249.72 | 69 | 63367 | -244.31 | 89 | 30916 | -234.50 |
| 10 | 69662 | -285.47 | 30 | 4112 | -262.02 | 50 | 1024 | -249.72 | 70 | 61741 | -244.15 | 90 | 9207 | -233.73 |
| 11 | 10698 | -284.98 | 31 | 33265 | -261.81 | 51 | 25006 | -249.53 | 71 | 38096 | -243.77 | 91 | 68722 | -232.30 |
| 12 | 52557 | -283.05 | 32 | 52699 | -261.65 | 52 | 69726 | -248.87 | 72 | 56416 | -242.87 | 92 | 45520 | -231.08 |
| 13 | 48801 | -278.13 | 33 | 60793 | -261.45 | 53 | 11011 | -248.46 | 73 | 32513 | -242.72 | 93 | 25174 | -230.93 |
| 14 | 21275 | -277.18 | 34 | 51288 | -260.77 | 54 | 46364 | -248.46 | 74 | 30391 | -240.70 | 94 | 54940 | -230.69 |
| 15 | 37809 | -277.11 | 35 | 31882 | -259.70 | 55 | 42611 | -248.42 | 75 | 70928 | -240.14 | 95 | 35000 | -230.63 |
| 16 | 46786 | -275.91 | 36 | 3106 | -258.03 | 56 | 48686 | -248.30 | 76 | 58317 | -239.94 | 96 | 55393 | -230.37 |
| 17 | 67241 | -275.24 | 37 | 66637 | -257.46 | 57 | 33931 | -248.14 | 77 | 70228 | -239.78 | 97 | 36565 | -229.46 |
| 18 | 38354 | -275.21 | 38 | 51163 | -257.08 | 58 | 5170 | -247.90 | 78 | 15567 | -238.99 | 98 | 47262 | -228.95 |
| 19 | 67002 | -274.68 | 39 | 30529 | -256.92 | 59 | 3235 | -247.31 | 79 | 51106 | -238.69 | 99 | 45609 | -228.28 |
| 20 | 30353 | -274.47 | 40 | 52148 | -256.68 | 60 | 844 | -246.87 | 80 | 33656 | -238.49 | 100 | 67384 | -227.99 |

Table 1 shows the binding energy value of 100 compounds with the most excellent binding strength as a result of the virtual screening.

Example 2: Fixation of Integrin αvβ3 Receptor on Substrate

An integrin αvβ3 receptor microarray (Integrin-tagged Protein Chip) was constructed by spotting the integrin αvβ3 (Chemicon, Temecula, Canada) on the surface of ProteoChip™ (Proteogen Inc., Seoul, Korea) with Microarrayer (CM-1000; Proteogen, Inc., Seoul, Korea). The integrin αvβ3 was diluted with a phosphate-buffered saline (PBS) containing 10 mM of β-octylthioglucopyranoside, 1mM of $CaCl_2$, 1mM of $MgCl_2$, and 30 % glycerol solution to be the concentration of 100μg/Mℓ; spotted; reacted at 4 ˚C overnight; and then the residue integrin αvβ3 after binding was washed with the phosphate-buffered saline (0.5 % PBST) containing 0.5 % Tween-20; and then stored at 4 ˚C when it

is used.

Example 3: High-Speed-High-Throughput Screening for Integrin αvβ3-OPN Interaction Antagonist from Library

3-1) Interaction of Integrin αvβ3 and Osteopontin

In order to investigate an interaction of the integrin αvβ3 and the osteopontin, the integrin αvβ3-tagged protein chip prepared from Example 2 was blocked with 3 % BSA for 1 hour, and then washed with PBST three times. And then, Cy-5 fluorescent-marked osteopontin with the concentration range of 10 μg/Mℓ to 12 ng/ml was diluted with a phosphate-buffered saline (PBS) containing 10 mM of β-octyl-thio-gluco-pyranoside, 1mM of $CaCl_2$, 1mM of $MgCl_2$, 0.2 mM of $MnCl_2$, and 30 % glycerol solution and spotted on the integrin microarray to react. Especially, it is known that the rules of calcium, manganese, and magnesium are important to the binding of the integrin and osteopontin.

Fig. 2 is fluorescence scan image showing the interaction of Integrin αvβ3 and Osteopontin and a concentration-reaction curve of the interaction of Integrin αvβ3 and Osteopontin. Specifically, Fig. 3b is a graph showing the result of measuring the relation between logs of the reactive fluorescence intensity of the osteopontin spot and Cy-5 fluorescent-marked fibronectin concentration.

From Fig. 2, it could be known that the integrin αvβ3 was well worked with Cy-5-marked osteopontin. More than all, the osteopontin was saturated at the concentration of at least about 1 μg/Mℓ. As a result, it could be known that the integrin αvβ3-tagged protein chip was valid and suitable for screening the inhibitor of the binding between the integrin αvβ3 and the osteopontin.

3-2) Preparation of Mixed Solution of Library and Osteopontin

The osteonpontin was marked with the fluorescent material (Cy-5; Amersham Parmacia Biotech, Uppsala Sweden) and then each mixed solution was prepared by mixing the osteopontin (1 μg/Mℓ) and the compound library (50 mM) derived from a natural material obtained from Example 1 in order to experiment the binding reaction of osteopontin-integrin. It was diluted with the phosphate-buffered saline (PBS) containing 10 mM of β-octyl-thio-gluco-pyranoside, 1mM of $CaCl_2$, 1mM of $MgCl_2$, 0.2 mM of $MnCl_2$, and 30 % glycerol solution to use.

3-3) Ultra-High-Speed-High-Throughput Screening for Inhibition of Binding of Osteopontin-Integrin αvβ3

The integrin αvβ3-tagged protein chip prepared from Example 2 was blocked with 3 % BSA for 1 hour, and then washed with PBST three times. And then, the mixed solution containing the osteopontin and each library prepared as mentioned above was spotted on the integrin αvβ3 receptor fixed on the surface of chip using Microarray (CM-1000; Proteogen, Inc., Seoul, Korea) under the conditions of the temperature of 30 °C and the humidity of 70 %, and then reacted for 1 hour. After washing with the washing solution (0.0 % PBST), it was dried with nitrogen, and then the inhibition ability of library was measured by analyzing the degree of ligand binding as a relative fluorescence intensity using a fluorescence laser scanner. As a result, it was certified that the compound effectively inhibited the binding reaction of the integrin αvβ3-osteopontin because the fluorescence intensities were significantly and relatively low in many libraries (see Fig. 3).

Fig. 3 is the experiment result for investigating the inhibition of the interaction of the integrin αvβ3 and osteopontin from the library.

For the above experiment, the osteopontin marked with the fluorescent material was used as a positive control and RGD peptide that was not marked was used as a negative control. The RGD was synthesized using an automatized peptide synthesizer from Peptron Company (Daejeon, Korea) to use for the experiment. It was reported that RGD (Amino acid sequence: GRGDSP) acts on the binding of the integrin αvβ3 and the osteopontin, and may play an inhibition rule as mention above.

The fluorescence intensity in Fig. 3 expresses rainbow colors. Originally, the result expresses single color, such as blue or red, but in the case of the above experiment, the software of the device gives the color changed according to the fluorescence intensity because the fluorescence intensity was not easily distinguished if the color was single.

The fluorescence intensity appears from the strongest fluorescence intensity in the order of white, red, orange, yellow, green, and blue. From the result of Fig. 2, it could be found that the color was white or red when reacting only with the fluorescent-marked osteopontin so that the binding between the integrin αvβ3 and the osteopontin was presented. When experimenting with the peptide including RGD (GRGDSP) base sequence, the interaction between the integrin αvβ3 and the osteopontin was inhibited so that the integrin αvβ3 and the osteopontin was not bound thereby appearing blue that means the lowest fluorescence intensity.

Some of libraries inhibit the interaction between the integrin αvβ3 and the osteopontin so that the integrin αvβ3 and the osteopontin were not bound thereby appearing blue that means the lowest fluorescence intensity. Therefore, it was verified that the specific compound was the material that strongly inhibits the binding between the integrin αvβ3 and the osteopontin (see Fig. 3).

3-4) Evaluation of Inhibitor for Binding between Osteopontin-Integrin αvβ3

$IC_{50}$ values for verifying the binding degree of the osteopontin marked with Cy5 in the concentration of 1 mg/ml and the ingegrin αvβ3 fixed in the concentration of 100 mg/ml were obtained by treating three inhibitors found from Example 3-3 using the method used in Example 3-3, in which the three inhibitors were used by diluting two times from

100 mM to 1 mM per concentration (see Fig. 4).

Example 4: Observation of Biological Activity for Materials Screened Using Protein Chip

Myeloplasts collected from five-week mice (C57BL/6J) were added to 48-well plate and cultured in a differentiation medium [a-MEM (Invitrogen. co.), 10 % (v/v) Fetal bovine serum (FBS), 100 units/ml penicillin, 100μg/ml streptomycin] including 30 ng/ml of hM-CSF (R&D system Inc.) that is a macrophage differentiator for three days under the conditions of 37 ˚C, 95 % (v/v) air, and 5 % (v/v) $CO_2$ to be $4x10^4$ cell/well so that the macrophase derived from the myeloplast was obtained. In this situation, in addition to the concentration of 30 ng/ml hM-CSF, mouse RANKL that is an osteoclast differentiator was expressed in *E. coli;* it was purely separated and purified; it was added in the concentration of 100 ng/ml; and then it was continuously cultured under the same conditions to induce the cell differentiation to the osteoclast. After culturing for 4 days, the cell was fixed with 3.7 % formaldehyde for 15 minutes at room temperature; washed with distilled water twice; the staining solution made by mixing acetate, Fast Gargnet GBC base, naphtol AS-BI phosphate, sodium nitride, and tartrate in the ratio as disclosed in the instructions of Acid Phosphatase, Leukocyte (TRAP) KitTM (Sigma Co.) was added to be 200 μg/well; and then reacted at 37 ˚C for 20 minutes to stain the differentiated osteoclast. As shown in Fig. 6, the differentiation inhibition ability of osteoclast was analyzed by treating 10 mM of **IPS-02001, IPS-02002,** and **IPS-02003** that were the osteopontin inhibitors using the above experiments. As a result, it could be known that **IPS-02001** and **IPS-02002** have excellent inhibition ability for differentiating to the osteoclast, but **IPS-02003** has no inhibition ability.

Example 5: Observation of Biological Activity for Materials Screened Using Protein Chip

For Fig. 6, the mouse osteoclast ($2X10^4$/well) was added to 48 well plate; M-CSF (30 ng/ml) and RANKL (200 ng/ml) were added at the same time; and then 10 mM of each of C01, CO2, and G10 that are an osteopontin inhibitor was treated to analysis the inhibition ability for differentiating to an osteoclast. As a result, it could be known that C01 and CO2 have excellent inhibition ability for differentiating to the osteoclast, but G10 has no inhibition ability.

Example 6: Mouse Calvaria Model

In order to investigate the inhibition ability of born destruction effect derived by RANKL or LPS in the real mice, LPS (12.5mg/kg) or RANKL (2mg/kg) were treated to skull calvaria of 7-week mouse to induce the born destruction; and then 20 mg/kg of the drug was treated for 4 days everyday. On 5 days, the mice calvaria were extracted; the cross-section of the calvaria was stained with H&E Staining to identify; and then the inhibition degree of the born destruction effect was analyzed using Image-pro Plus 4.5 (Media Cybernetics, Inc.).

It was verified that the IPS-02001 has the effect on inhibiting the born destruction effect due to LPS *in vivo* through the experiment for restoring the born cell destruction as follows: Lipopolysaccharide (10 mg/kg) that is an inflammatory inducible material that can destruct the born cell was treated to the mouse skull to induce an osteoporosis by artificial; and then IPS-02001 (10 mg/kg) was treated thereto (see Fig. 7).

## Claims

1. A method for screening an osteopontin inhibitor, comprising:

   a) screening a starting material for a protein chip screening by performing a molecular docking simulation to the whole files of compound library;
   b) preparing a mixture by mixing the screened starting material with an osteopontin and a fluorescent material-marked osteonpontin;
   c) adding the mixture on an integrin αvβ3 receptor fixed to the protein chip; and
   d) analyzing the degree of binding.

2. The method of claim 1, wherein a screening algorism for the docking calculation uses an alpha triangle process.

3. The method of claim 1, wherein the fluorescent material is at least one selected from the group consisting of Cy3 (Green), Cy5 (Red), FITC (Green), Alexa, BODIPY, Rhodamine, and Q-dot.

4. The method of claim 3, wherein the fluorescent material is Cy5.

5. An osteopontin inhibitor comprising at least one compound selected from the group consisting of the following Chemical Formula 4 and Chemical Formula 3 obtained form the method for screening according to any one of claims 1 to 3, a derivative thereof, or salt thereof:

[Chemical Formula 4]

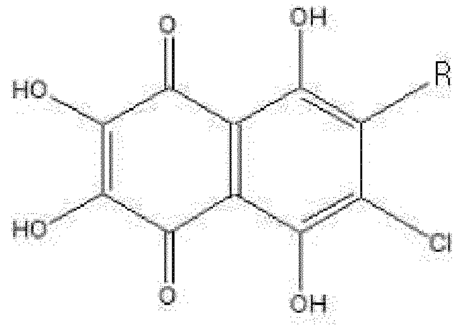

(In the above Chemical Formula 4,
R is H or Cl.)

[Chemical Formula 3]

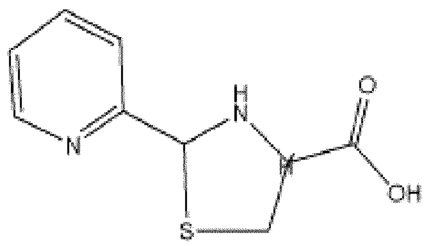

(IPS-02003) : 2-Pyridin-2-yl-thiazolidine-4-carboxylic acid

6. An osteopontin inhibitor comprising at least one compound selected from the group consisting of the following Chemcial Formula 4 and Chemical Formula 3, a derivative thereof, and salt thereof:

[Chemical Formula 4]

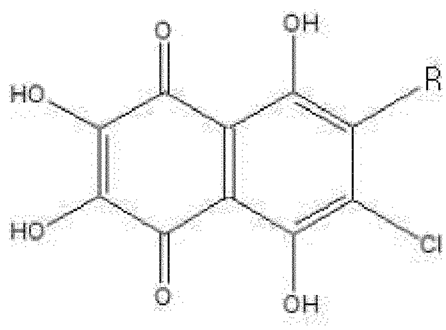

(In the above Chemical Formula 4,
R is H or Cl.)

[Chemical Formula 3]

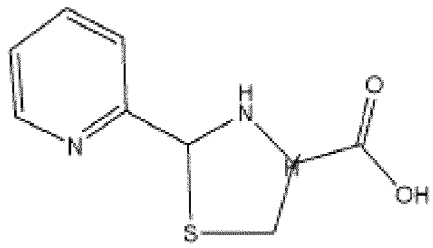

(IPS-02003) : 2-Pyridin-2-yl-thiazolidine-4-carboxylic acid

7. A composition for treating or preventing an osteoporosis, a rheumatoid arthritis, or a periodontal disease, comprising at least one compound selected from the group consisting of the following Chemical Formula 4 and Chemical Formula 3 obtained from the method for screening according to any one of claims 1 to 3, a derivative thereof, or salt thereof as an effective component:

[Chemical Formula 4]

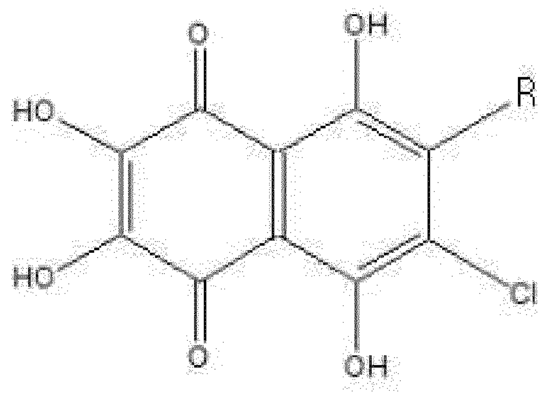

(In the above Chemical Formula 4,
R is H or Cl.)

[Chemical Formula 3]

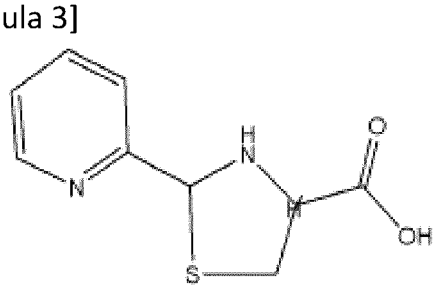

(IPS-02003) : 2-Pyridin-2-yl-thiazolidine-4-carboxylic acid

8. A composition for treating or preventing an osteoporosis, a rheumatoid arthritis, or a periodontal disease, comprising at least one compound selected from the group consisting of the following Chemical Formula 4 and Chemical Formula 3, a derivative thereof, or salt thereof as an effective component:

[Chemical Formula 4]

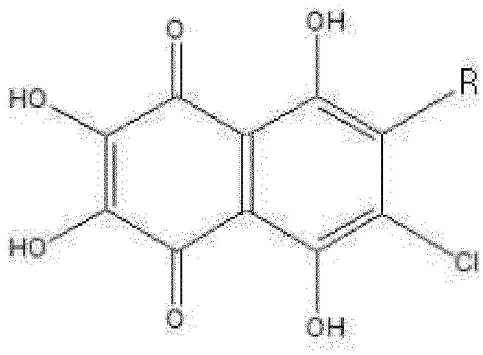

(In the above Chemical Formula 4, R is H or Cl.)

[Chemical Formula 3]

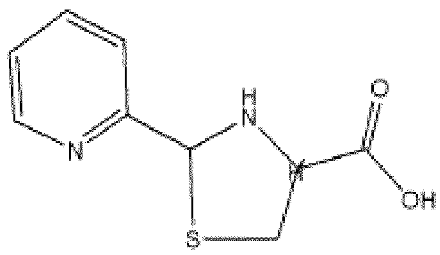

(IPS-02003) : 2-Pyridin-2-yl-thiazolidine-4-carboxylic acid

[Fig. 1]

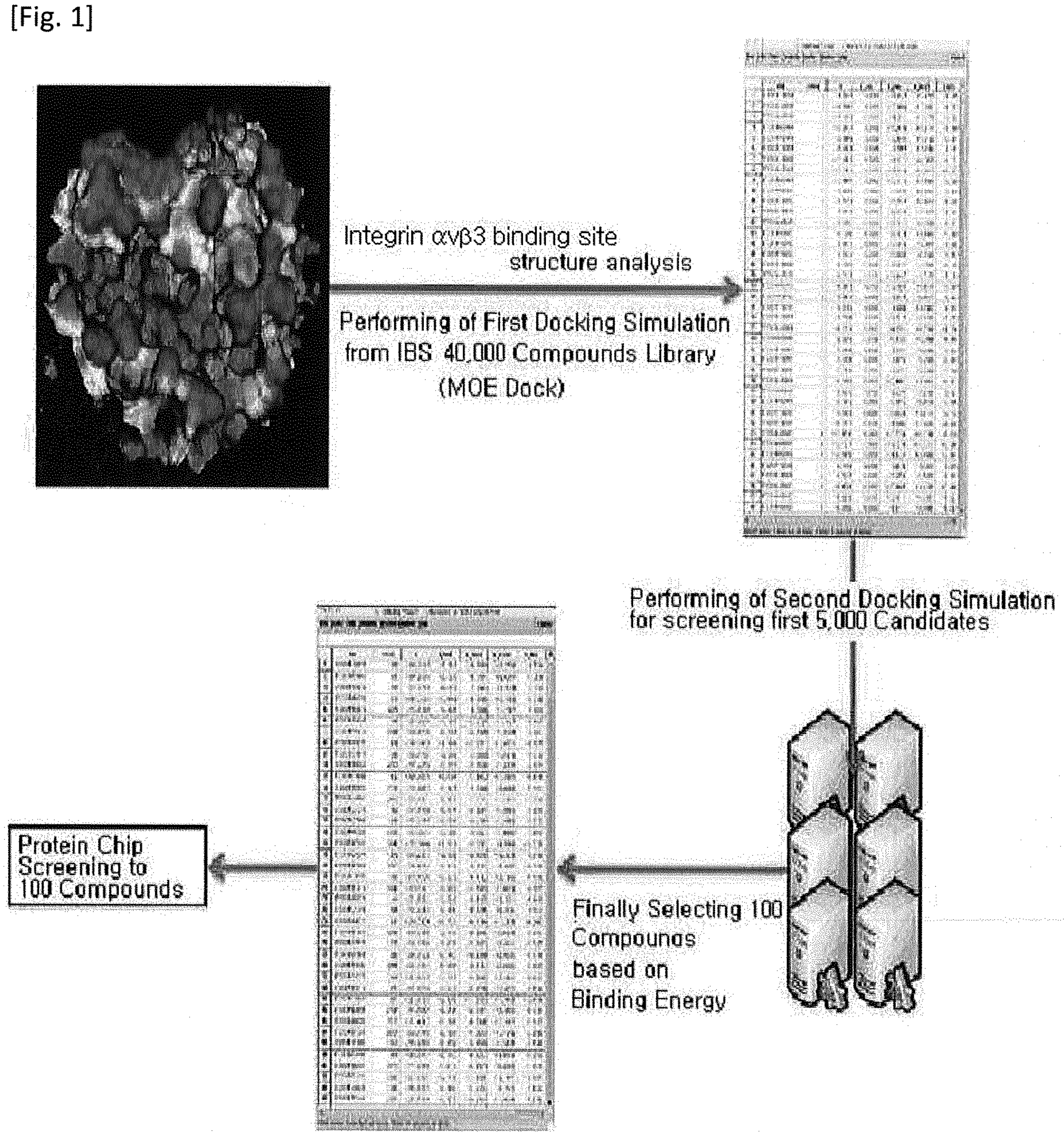
Integrin αvβ3 binding site
structure analysis
Performing of First Docking Simulation
from IBS 40,000 Compounds Library
(MOE Dock)
Performing of Second Docking Simulation
for screening first 5,000 Candidates
Finally Selecting 100
Compounds
based on
Binding Energy
Protein Chip
Screening to
100 Compounds

[Fig. 2]

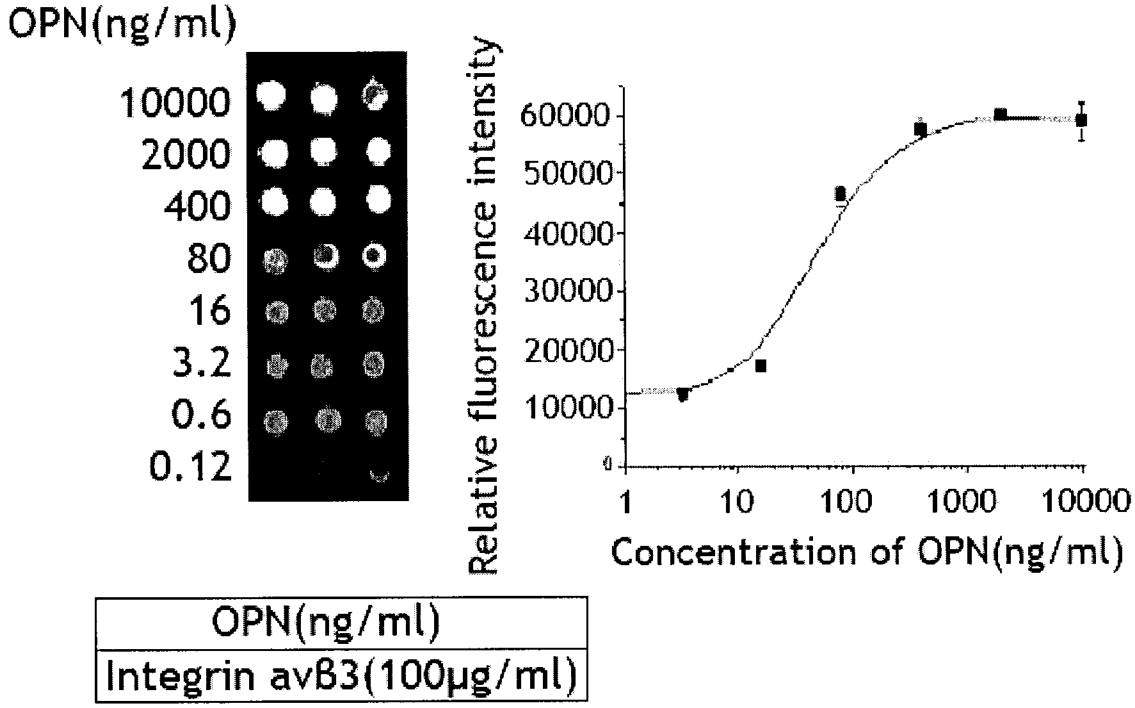
OPN(ng/ml)
10000
2000
400
80
16
3.2
0.6
0.12
Relative fluorescence intensity
60000
50000
40000
30000
20000
10000
0
1
10
100
1000
10000
Concentration of OPN(ng/ml)
OPN(ng/ml)
Integrin avß3(100µg/ml)

[Fig. 3]

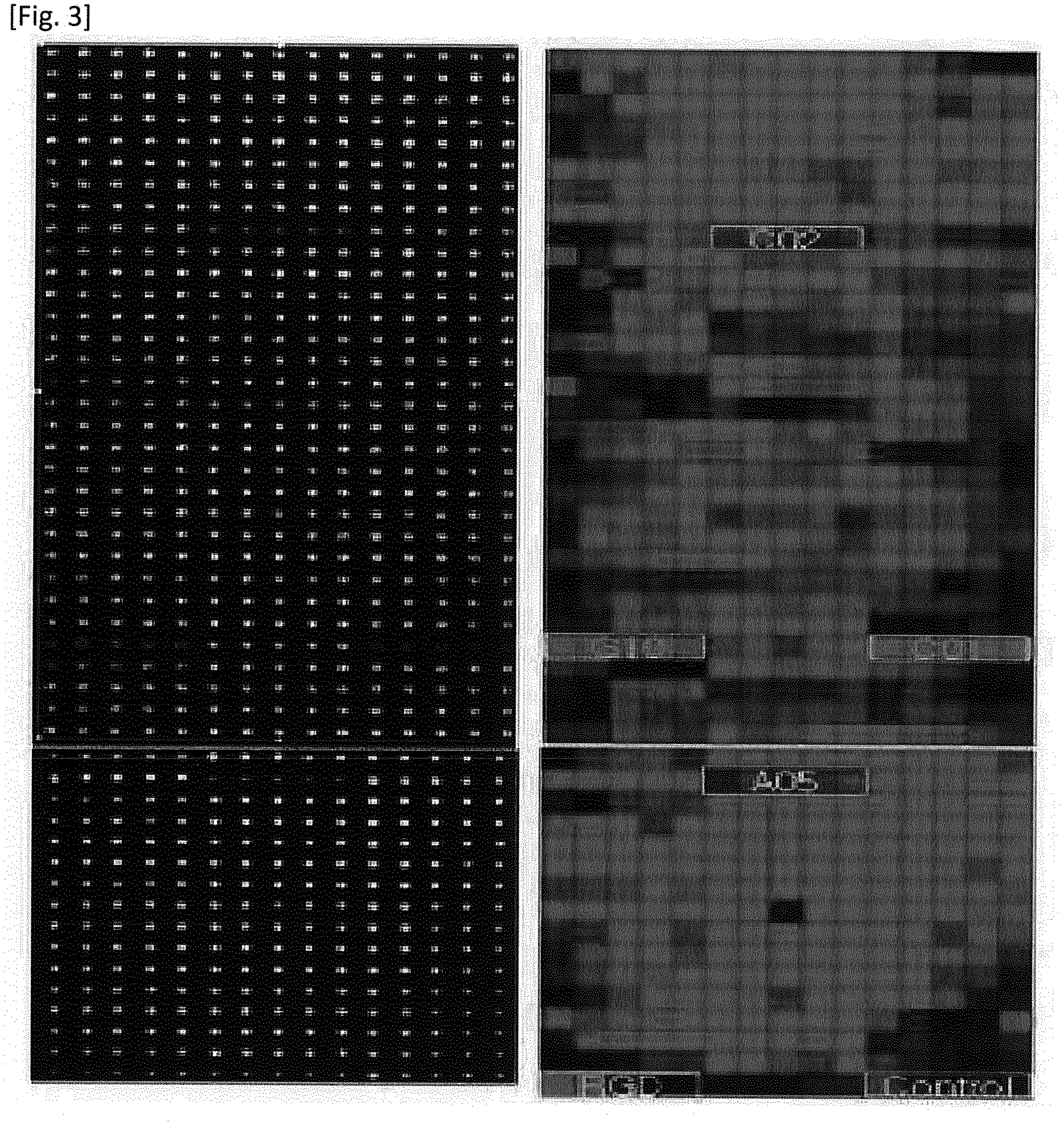

[Fig. 4]

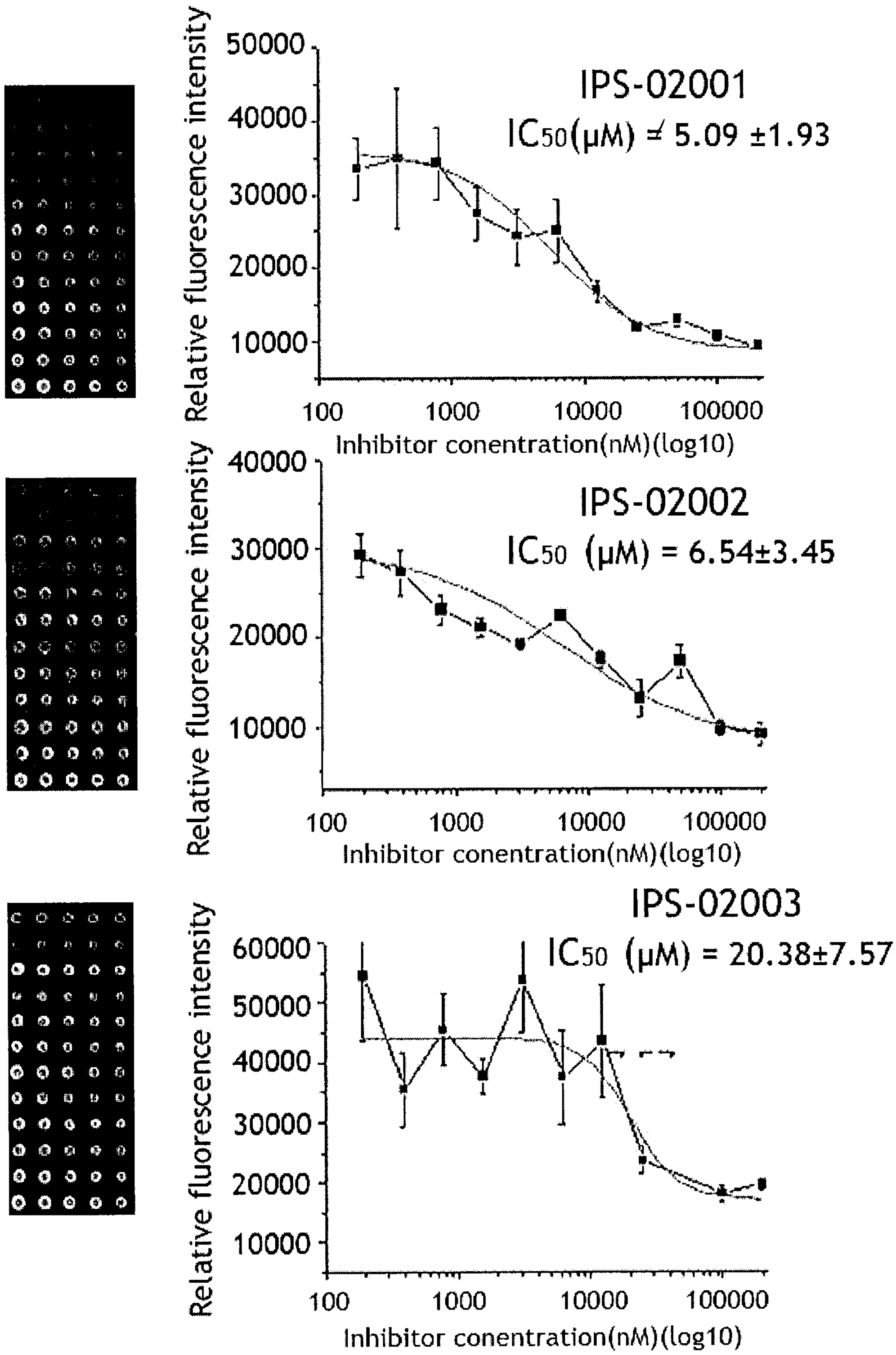
IPS-02001
IC50(μM) ≒ 5.09 ±1.93
Relative fluorescence intensity
50000
40000
30000
20000
10000
100
1000
10000
100000
Inhibitor conentration(nM)(log10)
IPS-02002
IC50 (μM) = 6.54±3.45
Relative fluorescence intensity
40000
30000
20000
10000
100
1000
10000
100000
Inhibitor conentration(nM)(log10)
IPS-02003
IC50 (μM) = 20.38±7.57
Relative fluorescence intensity
60000
50000
40000
30000
20000
10000
100
1000
10000
100000
Inhibitor conentration(nM)(log10)

[Fig. 5]

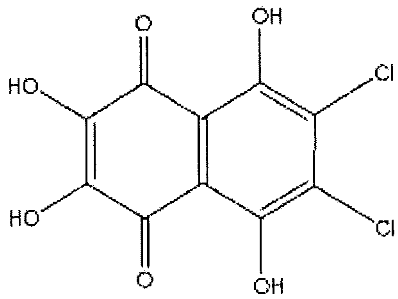

(IPS-02001) :
6,7-Dichloro-2,3,5,8-tetrahydroxy-[1,4]naphthoquinone

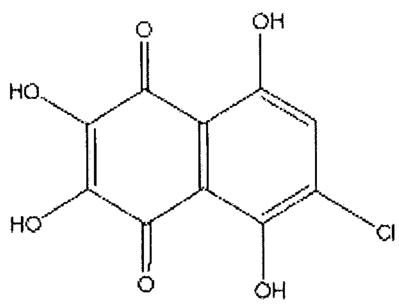

(IPS-02002) :
6-Chloro-2,3,5,8-tetrahydroxy-[1,4]naphthoquinon

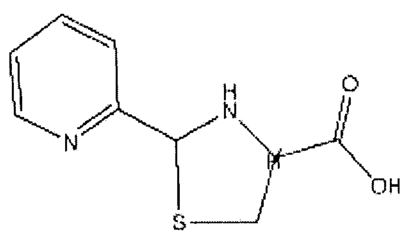

(IPS-02003) : 2-Pyridin-2-yl-thiazolidine-4-carboxylic acid

[Fig. 6]

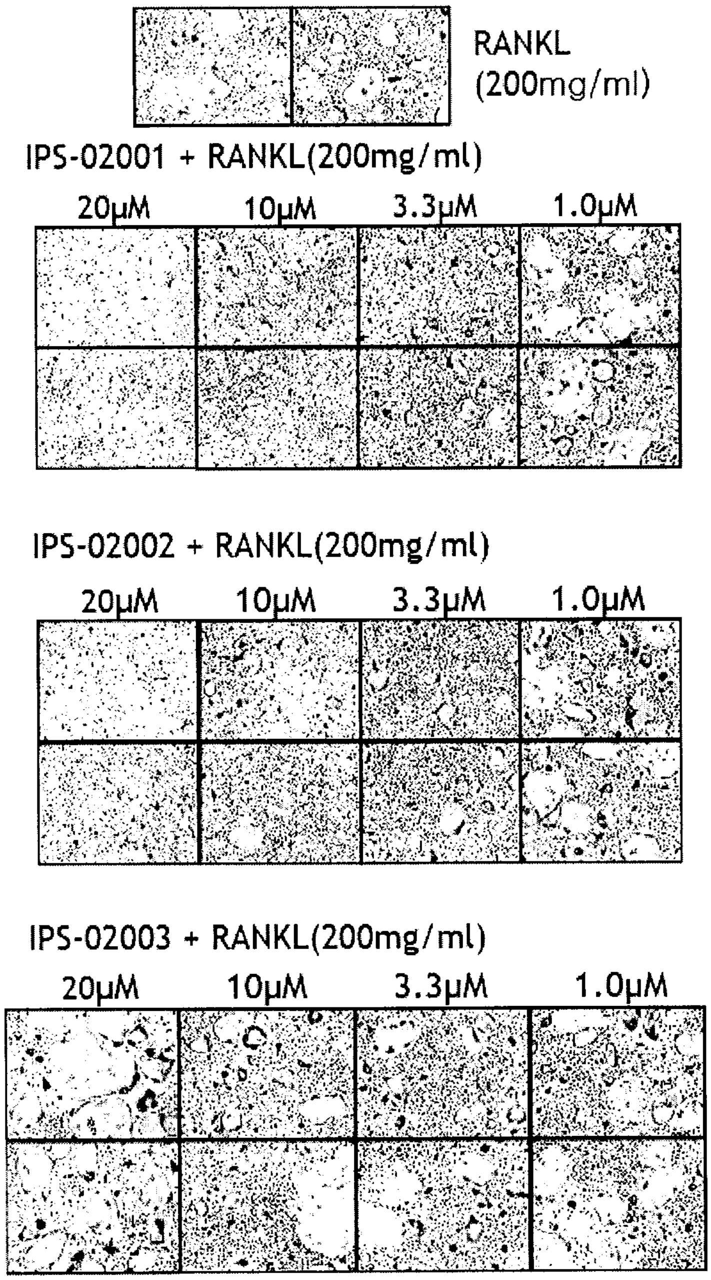
RANKL
(200mg/ml)
IPS-02001 + RANKL(200mg/ml)
20μM
10μM
3.3μM
1.0μM
IPS-02002 + RANKL(200mg/ml)
20μM
10μM
3.3μM
1.0μM
IPS-02003 + RANKL(200mg/ml)
20μM
10μM
3.3μM
1.0μM

[Fig. 7]

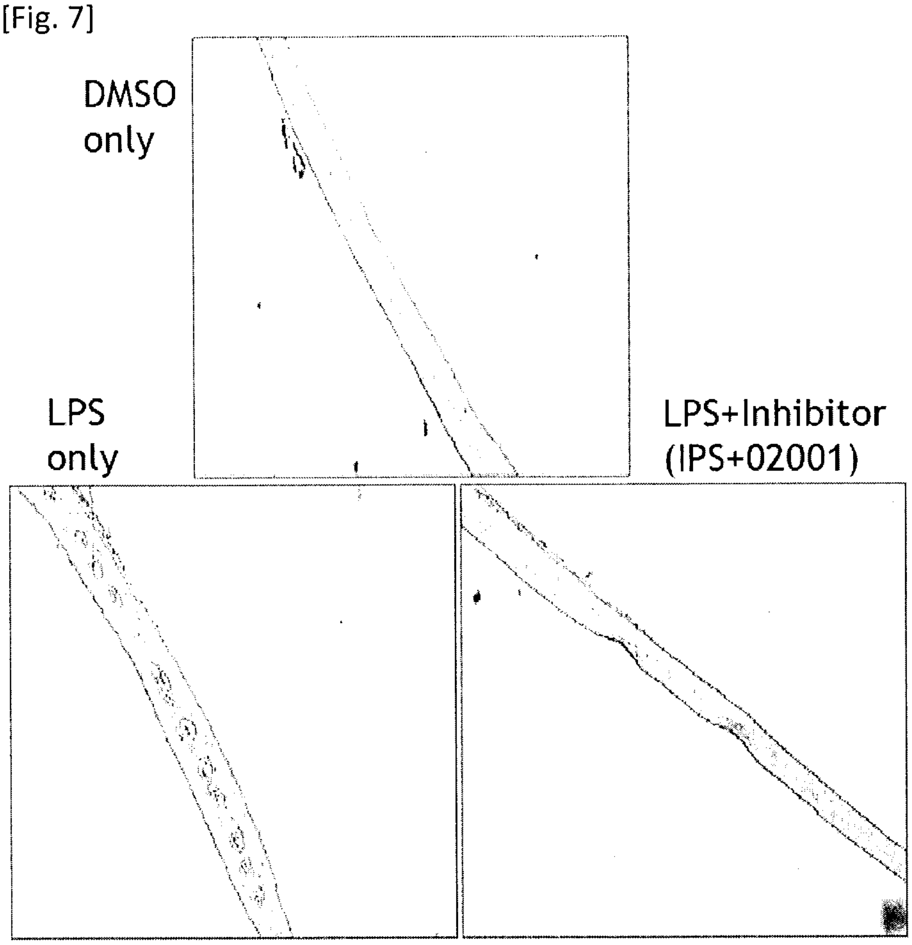

[Fig. 8]

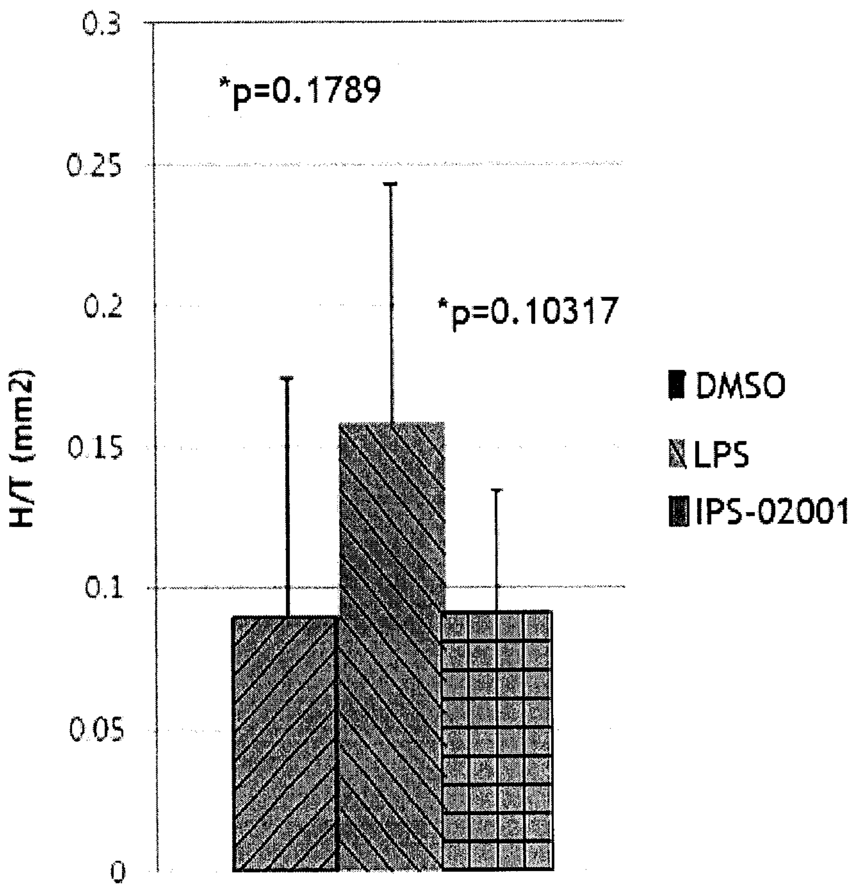

**INTERNATIONAL SEARCH REPORT**

International application No.
**PCT/KR2009/007114**

A. CLASSIFICATION OF SUBJECT MATTER

***G01N 33/15(2006.01)i, G01N 33/68(2006.01)i, G01N 33/52(2006.01)i***

According to International Patent Classification (IPC) or to both national classification and IPC

B. FIELDS SEARCHED

Minimum documentation searched (classification system followed by classification symbols)
G01N 33/15; A01K 67/027; A61B; A61K 38/17; A61K 38/54; G01N 33/574

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal)

C. DOCUMENTS CONSIDERED TO BE RELEVANT

| Category* | Citation of document, with indication, where appropriate, of the relevant passages | Relevant to claim No. |
|---|---|---|
| A | US 2007-0142281 A1 (YUKIHIKO SAEKI et al.) 21 June 2007<br>See abstract and claims 8-17 | 1-8 |
| A | US 2005-0250162 A1 (YUKIHIKO SAEKI et al.) 10 November 2005<br>See abstract and claims 1-7 | 1-8 |
| A | US 2002-0188962 A1 (DAVID T. DENHARDT et al.) 12 December 2002<br>See abstract. | 1-8 |
| A | WO 03-007794 A2 (CHILDREN'S MEDICAL CENTER CORPORATION) 30 January 2003<br>See abstract. | 1-8 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 JULY 2010 (12.07.2010) | **14 JULY 2010 (14.07.2010)** |

Name and mailing address of the ISA/KR
Korean Intellectual Property Office
Government Complex-Daejeon, 139 Seonsa-ro, Daejeon 302-701, Republic of Korea
Facsimile No. 82-42-472-7140

Authorized officer

Telephone No.

Form PCT/ISA/210 (second sheet) (July 2009)

INTERNATIONAL SEARCH REPORT
Information on patent family members

International application No.
PCT/KR2009/007114

| Patent document cited in search report | Publication date | Patent family member | Publication date |
|---|---|---|---|
| US 2007-0142281 A1 | 21.06.2007 | AU 2003-221092 A1 | 29.09.2003 |
| | | JP W020-030779 48A1 | 25.09.2003 |
| | | US 2005-0250162 A1 | 10.11.2005 |
| | | US 7745154 B2 | 29.06.2010 |
| | | WO 03-077948 A1 | 25.09.2003 |
| US 2005-0250162 A1 | 10.11.2005 | AU 2003-221092 A1 | 29.09.2003 |
| | | JP W020-030779 48A1 | 25.09.2003 |
| | | US 2007-0142281 A1 | 21.06.2007 |
| | | US 7745154 B2 | 29.06.2010 |
| | | WO 03-077948 A1 | 25.09.2003 |
| US 2002-0188962 A1 | 12.12.2002 | US 2002-188962 A1 | 12.12.2002 |
| | | US 6414219 B1 | 02.07.2002 |
| WO 03-007794 A2 | 30.01.2003 | AU 2002-254134 B2 | 01.06.2006 |
| | | CA 2440649-A1 | 19.09.2002 |
| | | CA 2454419-A1 | 30.01.2003 |
| | | EP 1372721 A2 | 02.01.2004 |
| | | EP 1372721 A4 | 02.04.2008 |
| | | EP 1417328 A2 | 12.05.2004 |
| | | IL159809D0 | 20.06.2004 |
| | | JP 04-295513 B2 | 17.04.2009 |
| | | JP 2004-537975 A | 24.12.2004 |
| | | JP 2005-218451 A | 18.08.2005 |
| | | JP 2005-519582 A | 07.07.2005 |
| | | JP 2005-519582 T | 07.07.2005 |
| | | JP 2009-017887 A | 29.01.2009 |
| | | WO 02-072759 A2 | 19.09.2002 |
| | | WO 02-072759 A3 | 19.09.2002 |
| | | WO 03-007794 A3 | 30.01.2003 |
| | | WO 0300-7794A3 | 12.09.2003 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Hiroyuki Yoshitake et al.** *PNAS,* 1999, vol. 96, 8156-8160
- **Kenji Yumoto et al.** *PNAS,* 2002, vol. 99, 4556-4561
- **Giachelli, C. M et al.** *Trends Cardiovasc Med,* 1995, vol. 3, 88-95
- **Ross, F.P. et al.** *J. Biol. Chem.,* 1993, vol. 268, 9901-9907
- **Khan SA. et al.** *Mol. Biol. Cell,* 2002, vol. 85, 728-736
- **Giachelli C. M. et al.** *Matrix biology,* 2000, vol. 19, 615-622
- **Ichiro Nakamura et al.** *J Bone Miner Metab,* 2007, vol. 25, 337-344
- **Dana D. Hu. et al.** *JBC,* 1995, vol. 28, 9917-9925
- **Boyle WJ. et al.** *Nature,* 2003, vol. 423, 337-42
- **Cathy J. Aitken et al.** *J. Cell. Biochem,* 2004, vol. 93, 896-903